# EUROPEAN PATENT SPECIFICATION

(11) **EP 2 628 467 B1**
(45) Date of publication and mention of the grant of the patent: **26.04.2017**
(21) Application number: 13155247.3
(22) Date of filing: 14.02.2013
(51) Int. Cl.: A61F 2/44, A61B 17/17, A61B 17/70, A61F 2/30, A61F 2/28

(54) **Intervertebral implant with improved shape of the fixing plate**
Zwischenwirbelimplantat mit verbesserter Form der Fixierungsplatte
Implant intervertébral présentant une forme améliorée de la plaque de fixation

(30) Priority: 17.02.2012 EP 12155953
(43) Date of publication of application: 21.08.2013
(73) Proprietor: Medacta International S.A., 6874 Castel San Pietro (TI) (CH)
(72) Inventor: Fiechter, Meinrad, 6874 Castel San Pietro (CH); Riva, Marco, 6874 Castel San Pietro (CH); Siccardi, Francesco, 6874 Castel Regina (CH)
(74) Representative: Vittorangeli, Lucia

(56) References cited:
- EP-A2- 1 834 608
- WO-A1-2010/107692
- WO-A2-2008/102174
- GB-A- 2 457 673
- US-A1- 2008 051 890
- US-A1- 2010 057 206
- US-A1- 2012 041 559

## Description

### Field of the invention

The present invention relates to the general field of orthopedic surgical implants.

In particular, the invention relates to an intervertebral implant permitting the fusion between two vertebral bodies of a vertebral column, used in the field of surgical spine treatment.

More specifically, the implant is preferably intended to be used as stand-alone intervertebral body fusion device for Anterior Lumbar Interbody Fusion (ALIF).

### Prior art

The ALIF approach is a surgical technique mainly used to operate a damaged disk in the lumbar area of the spine. The operation involves approaching the spine through an incision in the abdomen, removing the damaged disk and substituting it with a suitable intervertebral implant.

The intervertebral implant mainly comprises a hollow cage, which is inserted between the two vertebral bodies and filled with autogenic bone graft or the like in order to promote bone growth and eventual fusion of the two bodies.

Moreover, the implant comprises a fixing plate solidly attached to the hollow cage. The fixing plate may or may not protrude out of the intervertebral space and is fixed to the anterior sides of the two adjacent vertebral bodies, usually by means of two or more bone screws. The main function of the fixing plate and screws is that of improving primal stability of the whole implant.

A drawback of the ALIF approach lies in the fact that, above the fifth lumbar vertebra, the major blood vessels are located directly in front of the spine. Therefore, the surgeon has to perform a dissection of the vessels in order to determine a surgical access for the insertion of the intervertebral plate. This preliminary operation is time-consuming and challenging.

In view of the foregoing, the technical problem underlying the present invention is to provide an intervertebral implant which can be inserted and fixed on a patient's spine above the fifth lumbar vertebra, without the need of dissecting the blood vessels.

### Summary of the invention

The abovementioned technical problem is solved by an intervertebral implant as defined by claim 1.

As may be readily acknowledged by a person skilled in the art, the asymmetrical shape of the intervertebral implant makes it possible for the surgeon to adopt a new operative approach. Indeed, the intervertebral implant may be inserted in an oblique direction, e.g. at 45° with respect to the mid-sagittal plane, so that no dissection of the vessels in front of the vertebrae is required. Furthermore, all the bone screw may be inserted in the passageways adopting the same surgical angle and approach.

The fixing plate may advantageously feature a wedge-shaped portion on the lateral side opposite to that of the enlarged lateral portion.
Such a wedge-shaped portion is morphologically adapted to be inserted into the bone tissue of the patient, therefore easing the task of the surgeon
The anterior surfaces of the wedge-shaped portion and of the enlarged lateral portion may advantageously converge on an anterior edge of the fixing plate, said anterior surfaces sloping in a posterior direction from said anterior edge to their lateral edges.
In order to minimize the bulk of the implant, the wedge-shaped portion may have the same height of the fusion cage.

The passageways may comprise at least an upper passageway and at least a lower passageway, symmetrically disposed with respect to a transverse plane.

In particular, the passageways may comprise two upper passageways and two lower passageways. Alternatively, the passageways may comprise only a single upper passageway and only a single lower passageway.

Preferably, the angle between said passageways and the median plane is comprised between 30° and 60°.

In an embodiment of the intervertebral implant according to the present invention, the fixing plate may be integrally formed with the fusion cage.

Alternatively, the intervertebral implant may comprise a fastening mechanism for fastening the fixing plate on the fusion cage.

The fastening mechanism may alternatively comprise: a centering pin cooperating with a centering hole along with protrusions cooperating with a rotational slideway; protrusions cooperating with a vertical slideway; protrusions cooperating with a horizontal slideway; or a plurality of centering pins cooperating with centering holes and a single fastening screw for holding the fusion cage and the fixing plate together.

Further features and advantages of the intervertebral implant according to the invention shall be made clearer by the description, given herein below, of a several embodiments described by way of non-limiting examples with reference to the accompanying drawings.

### Brief description of the drawings

Figure 1 shows a perspective view of a first embodiment of an intervertebral implant according to the invention;
Figure 2 shows a perspective view of the intervertebral implant of figure 1 with its fixing plate and fusion cage detached;

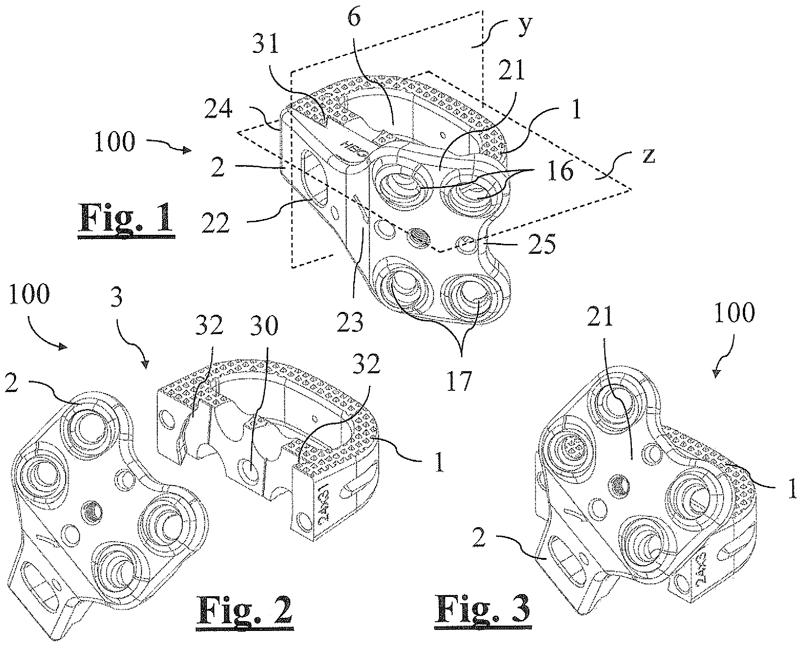

Figure 2 shows a perspective view of the intervertebral implant of figure 1 in an intermediate mounting configuration;

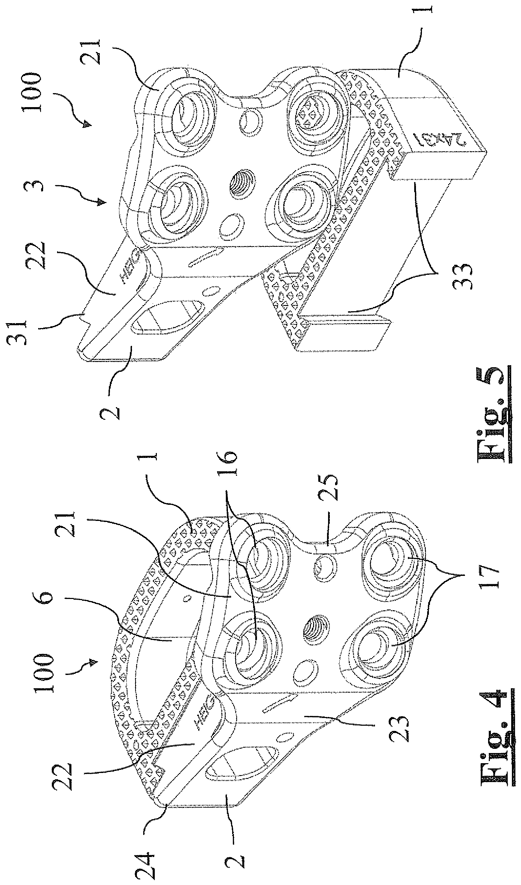

Figure 4 shows a perspective view of a second embodiment of an intervertebral implant according to the invention;
Figure 5 shows a perspective view of the intervertebral implant of figure 4 with its fixing plate and fusion cage detached;

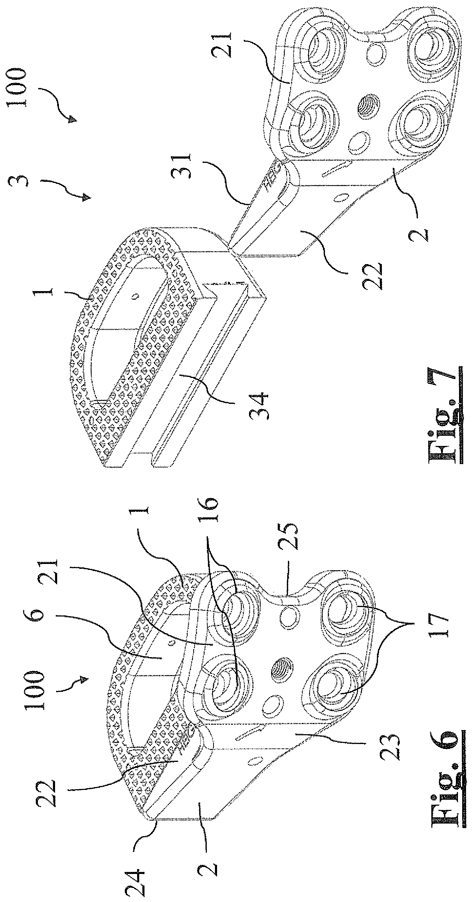

Figure 6 shows a perspective view of a third embodiment of an intervertebral implant according to the invention;
Figure 7 shows a perspective view of the intervertebral implant of figure 6 with its fixing plate and fusion cage detached;

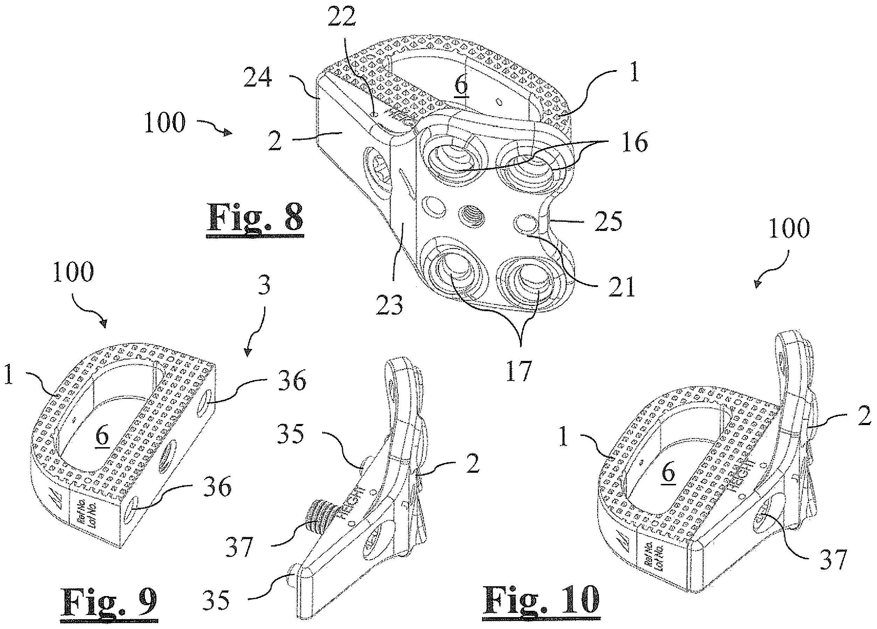

Figure 8 shows a perspective view of a fourth embodiment of an intervertebral implant according to the invention;
Figure 9 shows a perspective view of the intervertebral implant of figure 8 with its fixing plate and fusion cage detached;
Figure 10 shows a different perspective view of the intervertebral implant of figure 8;

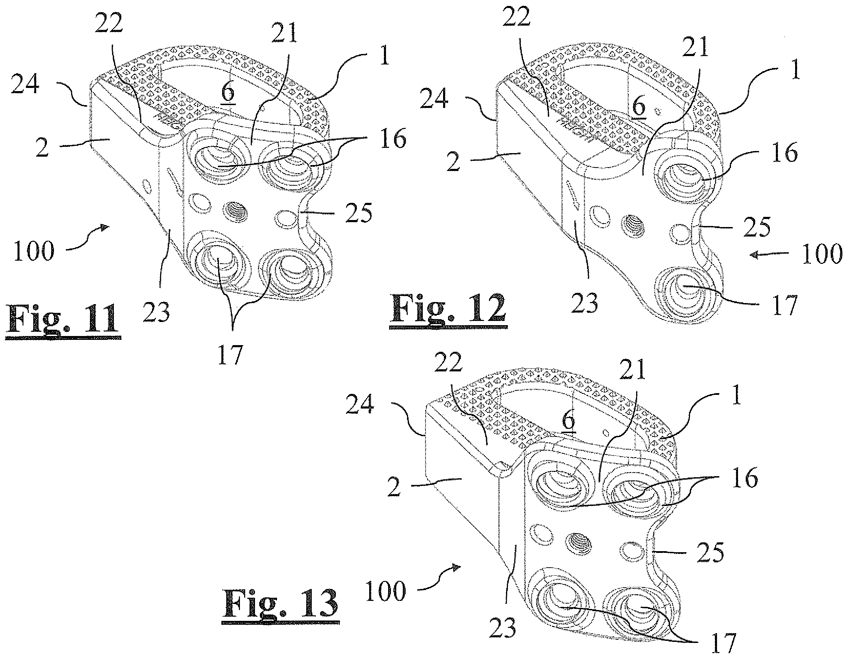

Figure 11 shows a perspective view of a fifth embodiment of an intervertebral implant according to the invention;
Figure 12 shows a perspective view of a sixth embodiment of an intervertebral implant according to the invention;
Figure 13 shows a perspective view of a seventh embodiment of an intervertebral implant according to the invention.

### Detailed description

Referring to figures 1-13, with 100 is globally shown an intervertebral implant, according to several embodiments of the present invention.

It should be noted that the same reference numerals have been used to identify the elements and parts which are similar or identical, either in shape or function, in the different embodiments of the invention.

A first embodiment of the intervertebral implant 100, shown in figures 1-3, comprises a fusion cage 1 coupled to a fixing plate 2 by means of a fastening mechanism globally referred as 3.

The fusion cage 1, intended to be inserted within the intervertebral space of a patient, features four walls of uniform height raising from a substantially rectangular base and enclosing a central hole 6.

The posterior side of the fusion cage 1, which is the side inserted between the vertebral bodies, exhibit rounded edges, while the anterior side, which has to be coupled with the fixing plate 2, has straight edges.

The central hole 6, extending in caudal-cranial direction, is intended to be filled with material for bone growth promotion, such as autogenic bone graft. The caudal and cranial cage surface are advantageously covered by a toothed pattern in order to improve primal stability.

The fusion cage 1 is made out of biocompatible material, for instance PEEK, CFRP or titanium, with or without a coating, for example a titanium or hydroxyapatite coating could be provided even if other coating may be used.

The fixing plate 2 has an asymmetrical structure. In order to describe its structure, two perpendicular planes should be preliminary introduced: a median plane y dividing the intervertebral implant 100 in two lateral asymmetrical halves, and a transverse plane z dividing the implant in cranial and caudal roughly symmetrical halves.

Indeed, the fixing plate features an enlarged lateral portion 21 protruding in both caudal, cranial and lateral directions with respect to the height and width of the fusion cage 1.

Said enlarged portion 21 has four passageways 16, 17 for housing bone screws. Two upper passageways 16 are positioned on the upper side - i.e. the side protruding in cranial direction - of the enlarged portion 21; two lower passageways 17 are symmetrically placed on the lower side.

It should be noted that all passageways 16, 17 converge toward the median plane of the intervertebral implant 100.

The enlarged portion itself 21 is basically a flat tab inclined with respect to the frontal side of the fusion cage 1. In particular, the enlarged portion 21 slopes from its lateral edge 25, protruding with respect to the lateral side of the fusion cage 1, toward an anterior edge 23 of the fixing plate 2.

On the opposite side with respect to the enlarged lateral portion 21, the fixing plate 2 features a wedge-shaped portion 22 having the same height of the fusion cage 1 and sloping from the anterior edge 23 toward the lateral edge 24 of the fixing plate 2. This lateral edge 24 does not protrude with respect to the lateral side of the fusion cage 1.

The fixing plate 2 is conveniently realized with a biocompatible material, for instance PEEK, CFRP, titanium or with another alternative material suitable for implantable medical devices.

The fastening mechanism 3 provided for fastening the fixing plate 2 to the fusion cage 1 comprises a centering hole 30 of the fusion cage and a centering pin of the fixing plate 2 engaging therein. The cooperation of the two features promotes a rotation of the fixing plate 2 on the fusion cage 1; the engagement of dove-shaped protrusions 31 of the fixing plate 2 in a rotational slideway 32 defined in the fusion cage provides a further guide and a stop for said rotational mounting.

A second embodiment, depicted in figures 4-5, basically shares the same features of the previously described embodiment.

However, the fastening mechanism 3 differs from the previous one in that a vertical slideway 33 substitutes the rotational slideway 32; the centering hole 30 and pin are no longer required.

A third embodiment, depicted in figures 6-7, basically shares the same features of the previously described embodiments.

Here again, the only difference lies in the fastening mechanism, which employs a horizontal slideway 34.

A fourth embodiment, depicted in figures 8-10, basically shares the same features of the previously described embodiments.

Here again, a different fastening mechanism 3 is proposed. The fastening mechanism comprises two centering pins 35 housed in centering holes 36, while a single fastening screw 37 traverses the fixing plates 2 and engages in the fusion cage 1.

A fifth embodiment, depicted in figure 11, is substantially similar to those previously described, but differs in that the fixing plate 2 and the fusion cage 1 are preassembled. Therefore, no fastening mechanism 3 is provided.

A sixth embodiment, depicted in figure 12, features a slight variation in the fixing plate 2, which is provided with a single upper passageway 16 and a single lower passageway 17.

A seventh embodiment, depicted in figure 13, features an intervertebral implant 100 wherein the fixing plate 2 is monolithically formed with the fusion cage 1. The two elements are therefore made out of the same material.

Obviously a person skilled in the art, in order to meet specific needs, will readily acknowledge the possibility of changes and variations to the intervertebral implant described above, all comprised within the scope of protection defined by the following claims.

## Claims

1. Intervertebral implant (100) for the fusion between two vertebral bodies of a vertebral column comprising:
- a fusion cage (1), extending from a posterior side to an anterior side, adapted to be interposed between two adjacent vertebral bodies of a patient;
- a fixing plate (2), attached to the fusion cage (1) and having a plurality of passageways (16; 17) for the insertion of bone screws;
**characterized in that** a vertical median plane (y) of the intervertebral implant (100) divides the fusion cage in two symmetrical halves and the fixing plate (2) in two asymmetrical halves, said passageways (16; 17) being provided only on one of said asymmetrical halves, said passageways (16; 17) being inclined towards the median plane (y) in the postero-anterior direction; wherein the fixing plate (2, 2a), attached to the fusion cage (1) on an anterior and/or antero-lateral side, has an enlarged lateral portion (21) protruding in both caudal and cranial directions with respect to the height of the fusion cage (1), said passageways (16; 17) being provided on said enlarged lateral portion (21).

2. Intervertebral implant (100) according to claim 1, wherein the fixing plate has a wedge-shaped portion (22) on the lateral side opposite to that of the enlarged lateral portion (21).

3. Intervertebral implant (100) according to claim 2, wherein said passageways (16; 17) comprise at least an upper passageway (16) and at least a lower passageway (17), symmetrically disposed with respect to a transverse plane (Z).

4. Intervertebral implant (100) according to claim 3, wherein said passageways (16; 17) comprise two upper passageways (16) and two lower passageways (17).

5. Intervertebral implant (100) according to claim 4, wherein said passageways (16; 17) comprise only a single upper passageway (16) and only a single lower passageway (17).

6. Intervertebral implant (100) according to one of the previous claims, wherein the angle between said passageways (16; 17) and the median plane (y) is comprised between 30° and 60°.

7. Intervertebral implant (100) according to one of the previous claims, wherein the fixing plate (2) is integrally formed with the fusion cage (1).

8. Intervertebral implant (100) according to claim 7, wherein the fixing plate (2) is formed as a single component with the fusion cage (1).

9. Intervertebral implant (100) according to one of claims 1-6, comprising a fastening mechanism (3) for fastening the fixing plate (2) on the fusion cage (1).

10. Intervertebral implant (100) according to claim 9, wherein said fastening mechanism (3) comprises a centering pin cooperating with a centering hole (30), and protrusions (31) cooperating with a rotational slideway (32).

11. Intervertebral implant (100) according to claim 9, wherein said fastening mechanism (3) comprises protrusions (31) cooperating with a vertical slideway (33).

12. Intervertebral implant (100) according to claim 9, wherein said fastening mechanism (3) comprises protrusions (31) cooperating with a horizontal slideway (34).

13. Intervertebral implant (100) according to claim 9, wherein said fastening mechanism (3) comprises at least a fastening screw (37) for holding the fusion cage (1) and the fixing plate (2) together.

14. Intervertebral implant (100) according to claim 13, wherein the fastening mechanism (3) features a single fastening screw (37) and further comprises a plurality of centering pins (35) cooperating with centering holes (36).

## Patentansprüche

1. Zwischenwirbelimplantat (100) für die Fusion zwischen zwei Wirbelkörpern einer Wirbelsäule, umfassend:
- einen Fusionskorb (1), der sich von einer hinteren Seite zu einer vorderen Seite erstreckt, der angepasst ist, um zwischen zwei benachbarten Wirbelkörpern eines Patienten gelagert zu werden;
- eine Fixierungsplatte (2), die am Fusionskorb (1) angebracht ist und eine Vielzahl von Durchgängen (16; 17) zum Einsetzen von Knochenschrauben aufweist;
**dadurch gekennzeichnet, dass** eine vertikale Mittelebene (y) des Zwischenwirbelimplantats (100) den Fusionskorb in zwei symmetrische Hälften und die Fixierungsplatte (2) in zwei asymmetrische Hälften teilt, wobei die Durchgänge (16; 17) nur auf einer der asymmetrischen Hälften vorgesehen sind, wobei die Durchgänge (16; 17) gegenüber der Mittelebene, (y) in der hinteren/vorderen Richtung geneigt sind; wobei die Fixierungsplatte (2, 2a), die am Fusionskorb (1) an einer vorderen und/oder vorderen/seitlichen Seite angebracht ist, einen vergrößerten Seitenabschnitt (21) aufweist, der sowohl in kaudaler als auch kranialer Richtung in Bezug auf die Höhe des Fusionskorbs (1) herausragt, wobei die Durchgänge (16; 17) am vergrößerten Seitenabschnitt (21) vorgesehen sind.

2. Zwischenwirbelimplantat (100) nach Anspruch 1, wobei die Fixierungsplatte einen keilförmigen Abschnitt (22) auf der lateralen Seite entgegengesetzt zu derjenigen des vergrößerten Seitenabschnitts (21) aufweist.

3. Zwischenwirbelimplantat (100) nach Anspruch 2, wobei die Durchgänge (16; 17) wenigstens einen oberen Durchgang (16) und wenigstens einen unteren Durchgang (17) umfassen, die symmetrisch zu einer Querebene (Z) angeordnet sind.

4. Zwischenwirbelimplantat (100) nach Anspruch 3, wobei die Durchgänge (16; 17) zwei obere Durchgänge (16) und zwei untere Durchgänge (17) umfassen.

5. Zwischenwirbelimplantat (100) nach Anspruch 4, wobei die Durchgänge (16; 17) nur einen einzelnen oberen Durchgang (16) und nur einen einzelnen unteren Durchgang (17) umfassen.

6. Zwischenwirbelimplantat (100) nach einem der vorhergehenden Ansprüche, wobei der Winkel zwischen den Durchgängen (16; 17) und der Mittelebene (y) zwischen 30° und 60° liegt.

7. Zwischenwirbelimplantat (100) nach einem der vorhergehenden Ansprüche, wobei die Fixierungsplatte (2) einstückig mit dem Fusionskorb (1) ausgebildet ist.

8. Zwischenwirbelimplantat (100) nach Anspruch 7, wobei die Fixierungsplatte (2) als eine einzelne Komponente mit dem Fusionskorb (1) ausgebildet ist.

9. Zwischenwirbelimplantat (100) nach einem der Ansprüche 1-6, umfassend einen Befestigungsmechanismus (3) für die Befestigung der Fixierungsplatte (2) auf dem Fusionskorb (1).

10. Zwischenwirbelimplantat (100) nach Anspruch 9, wobei der Befestigungsmechanismus (3) einen Zentrierstift, der mit einem Zentrierloch (30) zusammenwirkt, und Vorsprünge (31) umfasst, die mit einer rotierenden Gleitführung (32) zusammenwirken.

11. Zwischenwirbelimplantat (100) nach Anspruch 9, wobei der Befestigungsmechanismus (3) Vorsprünge (31) umfasst, die mit einer vertikalen Gleitführung (33) zusammenwirken.

12. Zwischenwirbelimplantat (100) nach Anspruch 9, wobei der Befestigungsmechanismus (3) Vorsprünge (31) umfasst, die mit einer horizontalen Gleitführung (34) zusammenwirken.

13. Zwischenwirbelimplantat (100) nach Anspruch 9, wobei der Befestigungsmechanismus (3) wenigstens eine Befestigungsschraube (37) zum Zusammenhalten des Fusionskorbs (1) und der Fixierungsplatte (2) umfasst.

14. Zwischenwirbelimplantat (100) nach Anspruch 13, wobei der Befestigungsmechanismus (3) über eine einzelne Befestigungsschraube (37) verfügt und ferner eine Vielzahl von Zentrierstiften (35) umfasst, die mit Zentrierlöchern (36) zusammenwirken.

## Revendications

1. Implant intervertébral (100) pour la fusion entre deux corps vertébraux d'une colonne vertébrale comprenant :
- une cage de fusion (1), se prolongeant d'un côté postérieur à un côté antérieur, pouvant être interposée entre deux corps vertébraux adjacents d'un patient ;
- une plaque de fixation (2), fixée à la cage de fusion (1) et comportant une pluralité de voies de passage (16 ; 17) pour l'insertion des vis à os ;
**caractérisé en ce qu'**un plan médian vertical (y) de l'implant intervertébral (100) divise la cage de fusion en deux moitiés symétriques et la plaque de fixation (2) en deux moitiés asymétriques, lesdites voies de passage (16 ; 17) étant pourvues uniquement sur une desdites moitiés asymétriques, lesdites voies de passage (16 ; 17) étant inclinées vers le plan médian (y) dans la direction postéro-antérieure ; dans lequel la plaque de fixation (2, 2a), fixée à la cage de fusion (1) sur un côté antérieur et/ou antéro-latéral, comporte une partie latérale élargie (21) dépassant à la fois dans les directions caudale et crâniale par rapport à la hauteur de la cage de fusion (1), lesdites voies de passage (16 ; 17) étant prévues sur ladite partie latérale élargie (21).

2. Implant intervertébral (100) selon la revendication 1, dans lequel la plaque de fixation comporte une partie en forme de coin (22) sur le côté opposé à celui de la partie latérale élargie (21).

3. Implant intervertébral (100) selon la revendication 2, dans lequel lesdites voies de passage (16 ; 17) comprennent au moins une voie de passage supérieure (16) et au moins une voie de passage inférieure (17), disposées symétriquement par rapport à un plan transversale (Z).

4. Implant intervertébral (100) selon la revendication 3, dans lequel lesdites voies de passage (16 ; 17) comprennent deux voies de passage supérieures (16) et deux voies de passage inférieures (17).

5. Implant intervertébral (100) selon la revendication 4, dans lequel lesdites voies de passage (16 ; 17) comprennent uniquement une seule voie de passage supérieure (16) et uniquement une seule voie de passage inférieure (17).

6. Implant intervertébral (100) selon l'une des revendications précédentes, dans lequel l'angle entre lesdits voies de passage (16 ; 17) et le plan médian (y) est compris entre 30° et 60°.

7. Implant intervertébral (100) selon l'une des revendications précédentes, dans lequel la plaque de fixation (2) est formée de façon intégrale avec la cage de fusion (1).

8. Implant intervertébral (100) selon la revendication 7, dans lequel la plaque de fixation (2) est formée d'un seul tenant avec la cage de fusion (1).

9. Implant intervertébral (100) selon l'une des revendications de 1 à 6, comprenant un mécanisme d'attache (3) servant à attacher la plaque de fixation (2) sur la cage de fusion (1).

10. Implant intervertébral (100) selon la revendication 9, dans lequel ledit mécanisme d'attache (3) comprend une cheville de centrage, coopérant avec un orifice de centrage (30), et des saillies (31) coopérant avec une glissière en rotation (32).

11. Implant intervertébral (100) selon la revendication 9, dans lequel ledit mécanisme d'attache (3) comprend des saillies (31) coopérant avec une glissière verticale (33).

12. Implant intervertébral (100) selon la revendication 9, dans lequel ledit mécanisme d'attache (3) comprend des saillies (31) coopérant avec une glissière horizontale (34).

13. Implant intervertébral (100) selon la revendication 9, dans lequel ledit mécanisme d'attache (3) comprend au moins une vis d'attache (37) servant à maintenir ensemble la cage de fusion (1) et la plaque de fixation (2).

14. Implant intervertébral (100) selon la revendication 13, dans lequel le mécanisme d'attache (3) présente une vis d'attache (37) unique et comprend de plus une pluralité de chevilles de centrage (35) coopérant avec orifices de centrage (36).
